# EUROPEAN PATENT APPLICATION

(11) **EP 2 980 582 A1**
(43) Date of publication of application: **03.02.2016**
(21) Application number: 14773104.6
(22) Date of filing: 28.03.2014
(51) Int. Cl.: G01N 30/88, B01J 39/20, B01J 39/26

(54) **CATION EXCHANGER FOR LIQUID CHROMATOGRAPHY, PROCESS FOR PRODUCING SAME, AND USE THEREOF**

(30) Priority: 29.03.2013 JP 2013075020
(71) Applicant: Tosoh Corporation, Syunan-shi, Yamaguchi 746-8501 (JP)
(72) Inventor: SAKO, Miyuki, Shunan-shi Yamaguchi 746-8501 (JP); MURANAKA, Kazuaki, Shunan-shi Yamaguchi 746-8501 (JP)
(74) Representative: TBK
(86) International application number: PCT/JP2014/059296
(87) International publication number: WO 2014/157670

(57) **Abstract**

To provide a cation exchanger for liquid chromatography having a high retention for an object to be separated or analyzed, a sufficient binding capacity for the object, a high resolution and mechanical strength, and a low operation pressure.

A cation exchanger for liquid chromatography, comprising non-porous particles and, immobilized to the surface thereof, a polyacrylic acid into which a dicarboxylic acid compound having an amino group is introduced by an amide bond.

## Description

### TECHNICAL FIELD

The present invention relates to a cation exchanger for liquid chromatography having a high retention for an object to be separated or analyzed, a sufficient binding capacity for the object, a high resolution and mechanical strength, and a low operation pressure.

### BACKGROUND ART

A cation exchanger for liquid chromatography is required to have the following properties.
(1) A high retention for an object substance (hereinafter, also referred to simply as "object") such as a protein so as to be less susceptible to concentration of a salt contained in a sample (retention).
(2) A sufficient binding capacity for an object so as to maintain separation performance for trace components (binding capacity).
(3) A high resolution and selection ability so as to improve accuracy of separation and analysis (resolution and selection ability).
(4) A mechanical strength to withstand a high operation flow rate so as to shorten a separation and analysis time (mechanical strength).
(5) A low operation pressure so as to be free from deterioration in an operation flow rate (operation pressure).

A cation exchanger for liquid chromatography is one obtained by introducing a cation exchange group (ligand) into particles to constitute the exchanger. As the particles, it has been proposed to use non-porous particles which are free from intra-particle diffusion (see Non-Patent Document 1), or particles having an average particle size of about 10 µm, for the purpose of improving the resolution and selection ability of the above (3).

As the average particle size becomes smaller, it has been proposed to use particles having an average particle size of at most 5 µm in recent years. In order to prevent the separation and analysis time from being prolonged by the use of such fine particles, it is necessary to exert a higher operation pressure, and therefore the mechanical strength of the above (4) required for particles becomes about 100 MPa.

Further, as the cation exchange group, e.g. a sulfonic acid group, a carboxy group and a phosphoric acid group have heretofore been known. Among them, a carboxy group is likely to undergo a change in dissociation degree depending on pH of eluent, exhibit a specific binding behavior such as formation of a hydrogen bond, and is thus likely to undergo a change in selectivity depending on elution conditions.

A strong cation exchanger such as a sulfonic acid type exchanger, which allows a hardly-separable sample to be separated, is widely used for e.g. separation of a protein isomer.

As methods of introducing a carboxylic acid group into particles, there have been known a method of copolymerizing a monomer such as acrylic acid or methacrylic acid with a crosslinkable monomer, a method of subjecting a halogenated alkyl carboxylic acid to desalting condensation with crosslinked particles having hydroxy groups by an alkali, and a method of introducing a compound having a polyfunctional carboxy group such as a polyacrylic acid, to particles having a functional group capable of being bonded with a carboxylic acid, such as an epoxy group, an amino group or a hydroxy group, by heating or using a condensation agent.

Non-porous particles (synthetic polymer particles) having synthetic polymers as a skeleton are ones capable of achieving the mechanical strength of (4) as well as the resolution and selection ability of the above (3). Accordingly, it is considered to employ a conventional method of introducing a cation exchange group to non-porous polymer particles. However, synthetic polymer particles are usually produced by using a copolymer of a monomer having a functional group for subsequently introducing cation exchange groups onto the particle surface and a polyfunctional unsaturated crosslinkable monomer for increasing the mechanical strength of the particles. Accordingly, if the polyfunctional unsaturated crosslinkable monomer is used in a large amount for increasing the mechanical strength of the particles, the amount of functional groups on the particle surface may decrease, a sufficient amount of cation exchange groups may not be introduced onto the particle surface, whereby the density of the cation exchange groups on the particle surface decreases. As a result, the retention for an object may be insufficient (the retention of the above (1) cannot be achieved), further, a surface area of such particles becomes small due to the non-porousness, whereby a binding capacity particularly for a biopolymer such as a protein or a nucleic acid may not be increased, the binding capacity of the above (2) may be impaired, and the elution peak of an object may be broad.

Accordingly, for the purposes of improving the retention of the above (1) and the binding capacity of the above (2), a method of grafting a vinyl polymer chain having a cation exchange ability so as to immobilize it onto the particle surface (see Patent Documents 1 to 4), or a method of immobilizing a water-soluble polymer into pores (see Patent Document 5) has been proposed. However, if the polymer chain is grafted to immobilize it to the particle surface, the operation pressure may increase, whereby an operation flow rate may be deteriorated, contrary to the requirement of the operation pressure of the above (5). Further, the method of immobilizing a water-soluble polymer into pores is not applicable to non-porous particles.

For the purpose of improving the binding capacity of the above (2) and the operation pressure of (5), a method of immobilizing onto the particle surface, a copolymer having a carboxylic acid group and a polymer having a functional group reactive with a carboxylic acid group, by partial crosslinking has been proposed (see Patent Document 6).

However, if this method is applied to fine particles having an average particle size of about 5 µm, an operation flow rate may be deteriorated, contrary to the requirement of the above (5). It is possible to lower the operation pressure by reducing a molecular weight of a polymer to be introduced, but the ion exchange capacity of a filler decreases, and therefore the retention of (1) and the binding capacity of (2) will hardly be achieved. Even when a low molecular weight ion exchange polymer having a molecular weight of about 5,000 is introduced, the operation pressure may increase by 1.5 to 2 times of the operation pressure before introduction, whereby the mechanical strength of (4) and the operation pressure of (5) may not sufficiently be improved, and a filler using such particles is also required to have further improved resolution and selection ability of (3).

### PRIOR ART DOCUMENTS

### PATENT DOCUMENTS

Patent Document 1: U.S. Patent 5,453,186
Patent Document 2: U.S. Patent 3,723,306
Patent Document 3: JP-B-57-23694
Patent Document 4: JP-B-57-58373
Patent Document 5: JP-A-2008-232764
Patent Document 6: JP-A-2002-306974

### NON-PATENT DOCUMENT

Non-patent Document 1: High-performance ion-exchange chromatography of proteins on non-porous ion exchangers, Journal of Chromatography A Volume 398, 1987, Page 327-334

### DISCLOSURE OF INVENTION

### TECHNICAL PROBLEM

In view of the above circumstances, the object of the present invention is to provide a cation exchanger for liquid chromatography, which achieves an improvement in the above properties (1) to (5), especially an improvement in the resolution and selection ability of (3).

### SOLUTION TO PROBLEM

The present inventors have conducted an extensive study and as a result, have found that a cation exchanger for liquid chromatography, comprising substantially non-porous particles and, immobilized to the surface thereof, a polyacrylic acid into which a dicarboxylic acid compound having an amino group is introduced, has a high retention for an object, a sufficient binding capacity for an object and a sufficient mechanical strength. They have also found that the cation exchanger for liquid chromatography shows a high separation performance and selection ability, particularly for a charge isomer such as an antibody, and has a low operation pressure, and they have accomplished the present invention.

That is, the present invention provides the following.
1. A cation exchanger for liquid chromatography, comprising non-porous particles and, immobilized to the surface thereof, a polyacrylic acid into which a dicarboxylic acid compound having an amino group is introduced by an amide bond.
2. The cation exchanger for liquid chromatography according to the above 1, wherein the non-porous particles are an inorganic base matrix of silica, zirconia or alumina, or an organic base matrix of a crosslinked polysaccharide or a vinyl monomer crosslinked polymer, having a volume average particle size of at most 5 µm.
3. The cation exchanger for liquid chromatography according to the above 2, wherein the organic base matrix is a copolymer of a monofunctional vinyl monomer and a polyfunctional vinyl monomer.
4. The cation exchanger for liquid chromatography according to the above 1, wherein the polyacrylic acid has a molecular weight of at least 5,000 and at most 20,000.
5. The cation exchanger for liquid chromatography according to the above 1, wherein the dicarboxylic acid compound having an amino group is aspartic acid or glutamic acid.
6. The cation exchanger for liquid chromatography according to the above 1, wherein the cation exchanger has an ion exchange capacity of from 10 to 50 µ-equivalent/mL.
7. A process for producing the cation exchanger for liquid chromatography as defined in any one of the above 1 to 6, comprising reacting a functional group of the non-porous particles with a carboxylic acid in the polyacrylic acid thereby to introduce the polyacrylic acid onto the surface of the non-porous particles, and reacting the particles having the polyacrylic acid introduced with the dicarboxylic acid compound having an amino group.
8. The process for producing the cation exchanger for liquid chromatography according to the above (7), wherein the non-porous particles are an inorganic base matrix of silica, zirconia or alumina, or an organic base matrix of a crosslinked polysaccharide or a vinyl monomer crosslinked polymer, having an average particle size of at most 5 µm.
9. The process for producing the cation exchanger for liquid chromatography according to the above 7 or 8, wherein water is used as a solvent.
10. The process for producing the cation exchanger for liquid chromatography according to any one of the above 7 to 9, wherein an amidation condensing agent or an alkali is further used as a catalyst for introducing the polyacrylic acid.
11. The process for producing the cation exchanger for liquid chromatography according to any one of the above 7 to 10, wherein an amidation condensing agent is further used as a catalyst for reacting the particles having the polyacrylic acid introduced with the dicarboxylic acid compound having an amino group.
12. The process for producing the cation exchanger for liquid chromatography according to the above (11), wherein the amidation condensing agent is 4-(4,6-dimethoxy-1,3,5-triazin-2-yl)-4-methylmorpholinium chloride.
13. A liquid chromatography column packed with the cation exchanger for liquid chromatography as defined in any one of the above 1 to 6.
14. A method of measuring a charge isomer of an antibody by ion exchange chromatography, comprising using the liquid chromatography column as defined in the above 13.
15. A method of measuring glycohemoglobin by ion exchange chromatography, comprising using the liquid chromatography column as defined in the above 13.

### ADVANTAGEOUS EFFECTS OF INVENTION

According to the cation exchanger of the present invention, it is possible to improve the properties of the above (1) to (5), and greatly improve the binding capacity of (2) and the resolution and selection ability of (3) in particular, as compared with conventional cation exchangers.

In a case where a vinyl monomer copolymer (crosslinked body) is used as the particles of the present invention, it is possible to achieve the mechanical strength capable of withstanding 100 MPa, whereby it is possible to improve the mechanical strength of (4), and further it is possible to improve the resolution and selection ability of (3) and the operation pressure of (5). In particular, according to the method of introducing a polyacrylic acid and then introducing a dicarboxylic acid having an amino group, it is possible to improve the operation pressure of (5) because of less increase in operation pressure by the introduction of polymer, and it is possible to improve the resolution and selection ability of (3) and the mechanical strength of (4) since measurement can be carried out at a higher flow rate.

### BRIEF DESCRIPTION OF DRAWINGS

Fig. 1 is chromatograms of Examples 1 to 3 and Comparative Examples 1 to 4.
Fig. 2 is chromatograms of Example 2 and Comparative Example 4.
Fig. 3 is chromatograms of Example 1 and Comparative Example 4.

### DESCRIPTION OF EMBODIMENTS

The cation exchanger for liquid chromatography of the present invention is one having non-porous particles as a base matrix. "Non-porous" in the present invention includes not only particles having no fine pores, but also particles having pores with a size that does not allow an object to be subjected to e.g. separation by a cation exchanger, to enter. In such a case, the average diameter of pores is preferably at most 20 A, more preferably at most 10 A. A method of controlling a size of pores in particles is known heretofore, as described in e.g. U.S. Patent 4,382,124.

The non-porous particles have a volume average particle size (D50) of preferably at most 5 µm, more preferably from 1.5 to 3.0 µm, for the purpose of improving the resolution and selection ability of (3) in particular.

The base matrix of the non-porous particles may, for example, be an inorganic base matrix (such as silica, zirconia or alumina), or an organic base matrix (such as a crosslinked polysaccharide, or a crosslinked product of a vinyl monomer such as acrylamide, an acryl ester or styrene). Among them, as the inorganic base matrix, a silica base matrix is preferred from the viewpoint of the cation exchange property of the base matrix, and as the organic base matrix, a crosslinked hydrophilic (meth)acrylic acid ester is preferred from the viewpoint of hydrophilicity and fine pores.

In particular, particles of the organic base matrix may be produced by a method of suspension polymerization of mixed fluid obtained by combining a monofunctional vinyl monomer (such as glycidyl methacrylate or vinylbenzylglycidyl ether) and a polyfunctional vinyl monomer (such as ethylene glycol dimethacrylate, polyethylene glycol dimethacrylate, glycerin polymethacrylate or divinylbenzene), as disclosed in e.g. JP-B-58-58026 or JP-A-53-90991, or a method of seed polymerization by combining the above monomers, disclosed in JP-A-2001-2716.

In the case of using non-porous particles of the organic base matrix, a copolymer of a monofunctional vinyl monomer and a polyfunctional vinyl polymer is preferred. For the purpose of improving (4), the proportion of the polyfunctional vinyl polymer is preferably at least 5 wt%, more preferably from 15 to 30 wt%. For the purpose of improving properties of the above (1) to (3), the proportion of the polyfunctional vinyl monomer is preferably at most 50 wt%, more preferably from 15 to 30 wt%, so as to introduce a sufficient amount of carboxylic acid groups and to secure a functional group, particularly preferably an epoxy group, for immobilizing a polyacrylic acid into which a dicarboxylic acid compound having an amino group is introduced.

For example, in the case of a copolymer of a monofunctional vinyl monomer having an epoxy group, such as glycidyl methacrylate, and a polyfunctional vinyl monomer, the epoxy group may be used as it is, or the epoxy group may be converted into a hydroxy group by hydrolyzing to make it undergo ring-opening, and followed by hydrophilization with a water-soluble polyhydric alcohol.

In the case of a copolymer having no epoxy group, e.g. a polyfunctional epoxy compound such as epichlorohydrin or ethylene glycol diglycidyl ether may be used to introduce an epoxy group, and then the copolymer is reacted in the same manner as the above.

The cation exchanger of the present invention is such that a carboxylic acid group as a cation exchange group is introduced onto the surface, and a polyacrylic acid into which a dicarboxylic acid compound having an amino group is introduced by an amide bond is immobilized to the surface thereof. The cation exchanger may, for example, be obtained by introducing a polycarboxylic acid polymer such as a polyacrylic acid by using an epoxy group at the surface, and introducing a dicarboxylic acid having an amino group to the polycarboxylic acid polymer introduced at the surface by using a condensing agent such as carbodiimide.

In order to improve the properties of the above (1) to (3) and (5), the polyacrylic acid into which a dicarboxylic acid compound having an amino group is introduced by an amide bond with a carboxy group of the polyacrylic acid, has a weight average molecular weight of preferably from 5,000 to 20,000, more preferably from 5,000 to 10,000. If the molecular weight is small, the effects of improving the properties of (1) to (3) become small, and if the molecular weight is too large, the effect of improving the property of (5) becomes small since the operation pressure increases.

The polyacrylic acid into which a dicarboxylic acid compound having an amino group is introduced by an amide bond, may be introduced to the particles after the dicarboxylic acid compound having an amino group is preliminarily introduced to the polyacrylic acid. From the viewpoint of introduction efficiency, it is suitable to employ a method of preliminarily introducing the polyacrylic acid to the particles, and then introducing the dicarboxylic acid compound having an amino group to the polyacrylic acid on the particle surface, by using the amidation condensing agent such as carbodiimide. According to this method, it is possible to obtain such an effect that the operation pressure is further lowered.

As the dicarboxylic acid compound having an amino group, it is suitable to use preferably an acidic amino acid such as aspartic acid or glutamic acid.

The dicarboxylic acid compound having an amino group may be introduced to the polyacrylic acid by using an amidation condensing agent for carboxylic acid. From the viewpoint of introduction efficiency, it is suitable to introduce the polyacrylic acid to the particles, and then directly introducing a dicarboxylic acid having an amino group thereto by using an amidation condensing agent such as 4-(4,6-dimethoxy-1,3,5-triazin-2-yl)-4-methylmorpholinium chloride and using an organic solvent/water mixed solvent in which the dicarboxylic acid having an amino group is soluble.

The organic solvent may, for example, be a water-soluble alcohol such as methanol, ethanol or propanol, dimethylsulfoxide, dimethylsulfonamide or chloroform.

Further, since the dicarboxylic acid having an amino group is hardly soluble in the organic solvent, it is suitable to use, as the amidation condensing agent, 4-(4,6-dimethoxy-1,3,5-triazin-2-yl)-4-methylmorpholinium chloride capable of condensation in a water solvent.

Moreover, since the dicarboxylic acid having an amino group is usually hardly soluble in an organic solvent, it is suitable to use a water-soluble carbodiimide such as 1-ethyl-3-(3-dimethylaminopropyl)carbodiimide hydrochloride (hereinafter also referred to as WSC) capable of condensation in a water solvent. In the case of using carbodiimide, in order to prevent polymerization of the dicarboxylic acid compound having an amino group, it is more suitable to convert a carboxy group in the polyacrylic acid into an active ester type such as N-hydroxysuccinimide, and then react the active ester with the dicarboxylic acid compound having an amino group.

A polyacrylic acid or a polyacrylic acid into which a dicarboxylic acid compound having an amino group is introduced, can be introduced to particles by imparting functional groups reactive with a carboxy group in the polyacrylic acid to particles thereby to bind them with the carboxy group.

The functional group reactive with a carboxy group may, for example, be an amino group, a hydroxy group or an epoxy group.

The above method of using an amidation condensing agent such as carbodiimide may, for example, be mentioned in the case of using particles having an amino group, a method of dehydration condensation by heating may, for example, be mentioned in the case of using particles having a hydroxy group, and a method of binding the groups in the presence of an alkali catalyst may, for example, be mentioned in the case of using particles having an epoxy group.

The amidation condensing agent in the case of using particles having an amino group may, for example, be 4-(4,6-dimethoxy-1,3,5-triazin-2-yl)-4-methylmorpholinium chloride or WSC. Further, in the case of using particles having an epoxy group, the alkali catalyst may, for example, be sodium hydroxide or pyridine.

The reaction may be carried out in a solvent in which the polyacrylic acid is soluble, but from the viewpoint of easiness and introduction efficiency, it is suitable to employ a method wherein the polyacrylic acid is dissolved in a volatile solvent, the particles are dispersed therein, the solvent is then distilled off by e.g. an evaporator, followed by heating thereby to bind the carboxylic acid in the polyacrylic acid to the functional group of the particles, since this method allows the polyacrylic acid to be introduced without necessarily using e.g. a catalyst.

In the method wherein the polyacrylic acid or the polyacrylic acid into which a dicarboxylic acid compound having an amino group is introduced by an amide bond and the particles having a functional group, are bound by heating after distillation off of the solvent, it is suitable to use a hydroxy group or an epoxy group as the functional group of the particles, from the viewpoint of mild reaction conditions.

Specifically, it is preferred that the polyacrylic acid or the polyacrylic acid into which a dicarboxylic acid compound having an amino group is introduced, in an amount of from 2 to 10 wt%, preferably from 5 to 10 wt% based on the particles, is dissolved in water, the resulting solution is then adjusted to have a pH of from 5 to 10, preferably from 6 to 8, a predetermined amount of the particles are dispersed therein, water is distilled off, followed by heating. The heating temperature is preferably from 160 to 200°C when the functional group of the particles is a hydroxy group, preferably from 110 to 150°C when the functional group is an epoxy group, and heating is preferably carried out for at least 1 hour.

The amount of the polyacrylic acid or the polyacrylic acid into which a dicarboxylic acid compound having an amino group is introduced, immobilized to the non-porous particles, is preferably such that the cation exchange capacity of the cation exchanger is preferably adjusted to be within a range of from 5 to 50 µm equivalent/mL (meq/L), preferably from 20 to 40 equivalent/mL (meq/L) for the purpose of improving (5). According to such control of the immobilization amount, it is possible to obtain a cation exchanger having a low operation pressure property.

When an epoxy group is used as the functional group of the particles, the recovery rate of a measurement sample tends to be low if an epoxy group which is not used for immobilizing the polyacrylic acid remains. In such a case, it is recommended to convert such a remaining epoxy group to a chemical structure having no adverse influences on ion exchange of a filler with a sample by using a compound reactive with the epoxy group. The compound reactive with the epoxy group may, for example, be water, a polyhydric alcohol having hydroxy groups such as ethylene glycol, glycerin or glucose, an amino acid to impart a betain structure such as glycin, alanine, aspartic acid, glutamic acid, lysine, arginine or taurine, or mercapto acetic acid having a thiol group.

The reaction pattern may not particularly be limited so long as the remaining epoxy group no longer interacts with the sample, therefore it is possible to use a compound to be introduced to a large excess amount of epoxy groups, and as the case requires, it is possible to use a catalyst under usual reaction conditions.

The cation exchanger of the present invention produced as the above comprises substantially non-porous particles and, immobilized to the surface thereof, a polyacrylic acid into which a dicarboxylic acid compound having an amino group is introduced, and it may be packed into e.g. a column for liquid chromatography so as to separate and purify a bio sample such as various proteins including an antibody and glycohemoglobin.

### EXAMPLES

Now, the cation exchanger of the present invention will be described in further detail with reference to Examples, but it should be understood that the present invention is by no means restricted thereto.

### [Preparation of non-porous base matrix]

Non-porous particles were produced by the method (seed polymerization method) disclosed in JP-A-2001-2716.

30 g of benzyl methacrylate and 1.5 g of 2-ethylhexyl mercaptoacetate were charged into a 500 mL three-necked flask and mixed with one another, and 300 g of deionized water was charged thereto. A magnetic stirrer was then put into the flask, and the flask was immersed in an oil bath at 72°C and provided with a nitrogen inlet tube, followed by stirring at 150 rpm. Separately, 0.9 g of potassium persulfate and 30 g of deionized water were weighed, and potassium persulfate was dissolved in a 50 mL container. After expiration of 30 minutes, an aqueous potassium persulfate solution was injected by a syringe through a rubber stopper provided in the three-necked flask. Soap free emulsion polymerization was carried out at a number of revolutions of 300 rpm. The polymerization was continued for 2 hours, and then agglomerates were removed to recover a seed solution. The solid content of the seed solution was 5.16 wt%, and the volume average particle size (D50) was 0.48 µm as measured with an electron microscope.

64 g of 2,3-epoxypropyl methacrylate, 16 g of ethylene dimethacrylate, 0.2 g of 2,2'-azobis(2,4-dimethylvaleronitrile) (tradename: V-65, manufactured by Wako Pure Chemical Industries, Ltd.) and 0.2 g of sodium dodecyl sulfate were weighed and into a 300 mL flask, and a magnetic stirrer was then put thereby to carry out mixing. Further, 100 mL of deionized water was added thereto, followed by emulsification by an ultrasonic homogenizer with stirring with the magnetic stirrer. 10.74 g (mass of solid content: 0.554 g) of the above seed solution prepared and 50 mL of a 4 wt% aqueous polyvinyl alcohol solution were added thereto, followed by thorough stirring for 1 minute, and the resulting mixture was left at rest. The mixture was left to stand for 30 minutes at room temperature, and then left at rest in a water bath at 60°C to carry out polymerization for 2 hours. Polymerization fluid thus obtained was filtrated by a glass filter, and washed with warm water, acetone and warm water in this order to obtain non-porous particles.

The non-porous particles obtained was charged Into a 500 mL separable flask, 300 mL of deionized water was added thereto, and the flask was immersed in an oil bath at 90°C and heated for 24 hours with stirring thereby to hydrolyze an epoxy group of the particles. The non-porous particles after hydrolyzing the epoxy group were observed by an electron microscope, and found to be uniform particles having a volume average particle size of 2.3 µm.

100 g of the non-porous particles obtained (water-suction dry), 103 g of epichlorohydrin and 100 g of deionized water were charged into a 500 mL separable flaks, and the flask was immersed in a water bath at 45°C, followed by stirring. Separately, 88 g of a 48 wt% aqueous sodium hydroxide solution was weighed and charged into a disposable syringe, which was then set in a syringe pump, and injected into a separable flask at a speed of 0.5 mL/min with stirring. Thereafter, reaction was continued for 2 hours to obtain a reaction product, which was then filtrated by a glass filter and washed with water and acetone in this order, followed by blow-drying to obtain epoxylated non-porous particles (epoxy equivalent: 840 µ equivalent/dry gel (g)). Further, the pore diameter of such particles with a molecular weight of at most 200 as calculated as polyethylene glycol, was at most 10 A.

### EXAMPLE 1

### <Introduction of polyacrylic acid>

0.75 g of polyacrylic acid (weight average molecular weight: 5,000, manufactured by Wako Pure Chemical Industries, Ltd.) was dissolved in 50 mL of deionized water, and a 2N aqueous sodium hydroxide solution was used to adjust pH to 6.8. 10 g of epoxylated non-porous particles were then charged therein and dispersed by an ultrasonic water bath, and the resulting dispersion was then charged into a 200 mL recovery flask and water was distilled off by an evaporator. Thereafter, the recovery flask was put in an oven set at 130°C and heated for 3 hours, and then 50 mL of deionized water was added to the recovery flask so as to disperse the particles. Filtration was carried out by a glass filter, and then the particles were washed with 0.1 mol/L phosphoric acid, 0.1 mol/L sodium hydroxide and deionized water in this order. 550 µ equivalent/dry gel (g) of unreacted epoxy groups were confirmed by a dioxane/hydrochloric acid method.

The particles dispersed in water were heated in an autoclave at 140°C for 3 hours thereby to add water to the remaining epoxy groups. The amount of epoxy groups was found to be at most 20 µ equivalent/dry gel (g) by a dioxane/hydrochloric acid method.

The particles were washed with a 0.1 mol/L hydrochloric acid and water in this order, and titrated with 0.01 N sodium hydroxide, whereupon 25 µ equivalent/mL of an ion exchange capacity was confirmed.

### <Introduction of aspartic acid>

4.5 g of the above deionized water suction dry particles were weighed and charged into a 40 mL centrifuge tube, and subjected to solvent substitution with a 20 mmol/L morpholinoethanesulfonic acid (hereinafter referred to as MES) (pH 5.5) by centrifugal filtration three times. 10 mL of a 20 mmol/L MES (pH 5.5) was further added thereto so as to disperse the particles. 540 mg of N-hydroxysuccinimide was further added thereto to carry out dispersion. 224 mg of WSC (manufactured by Peptide Institute, Inc.) was dissolved in 5 mL of a 20 mmol/L MES (pH 5.5), and the solution was charged to the dispersion, followed by stirring quickly. The resulting mixture was left at rest for 1 hour, and washed with a 20 mmol/L MES (pH 5.5) by centrifugal filtration 5 times. Thereafter, 20 mL of aspartic acid solution adjusted to have a concentration of 14 mmol/L and pH of 6.5 was then charged thereto so as to carry out dispersion, and then left at rest for 1 night. The ion exchange capacity of particles obtained was 27 µ equivalent/mL.

The cation exchanger obtained was packed in the form of a slurry into a liquid chromatography column having an inner diameter of 4.6 mm and a length of 100 mm, together with a 10% deionized water as dispersion fluid. Using the column obtained, liquid chromatography was carried out under the following conditions to measure the resolution of a monoclonal antibody. The operation pressure of the cation exchanger was an operation pressure at the time of feeding eluent A at a flow rate of 0.6 mL/min.

### LIQUID CHROMATOGRAPHY CONDITIONS

Eluent A: 20 mmol/L MES (pH 5.5)
Eluent B: 0.3 mol/L NaCl in 20 mmol/L MES (pH 5.5)
Gradient: 37 to 57% eluent B linear gradient, 60 minutes
Flow rate: 0.6 mL/min
Detection: UV 230 nm
Object: Monoclonal antibody, 1/10 diluent of eluent A, 10 µL

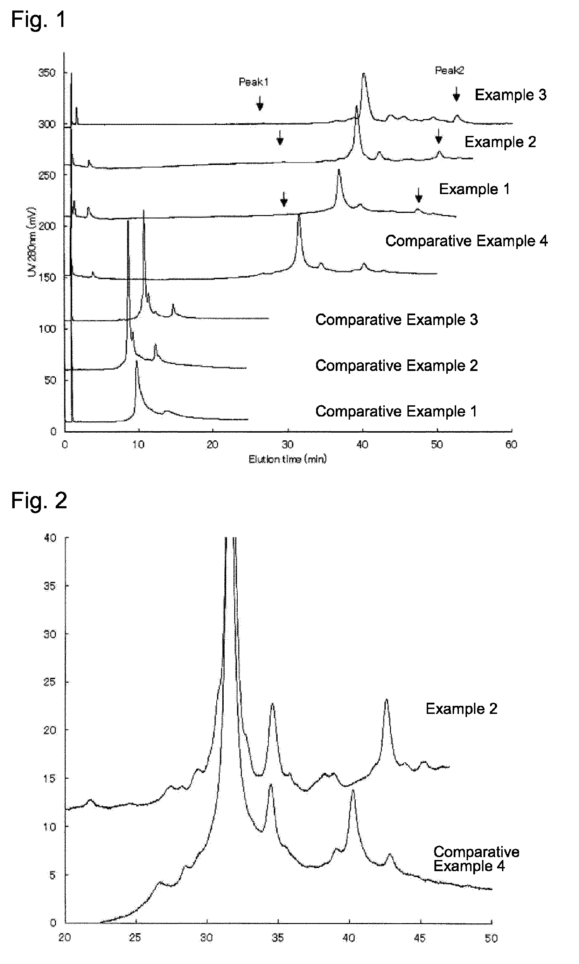

As shown in the chromatogram of Fig. 1, it was found that strong retention was achieved as compared with Comparative Examples, and it was also found that the separation performance between isomers was excellent. The elution time difference of from the elution peak 1 at an acidic side to the elution peak 2 at a basic side was 17.1 minutes, and broad selectivity was shown. The operation pressure was 13.0 MPa and was found to be low.

Further, liquid chromatography was carried out under the following condition, to measure the separation performance for glycohemoglobin.

### LIQUID CHROMATOGRAPHY CONDITIONS

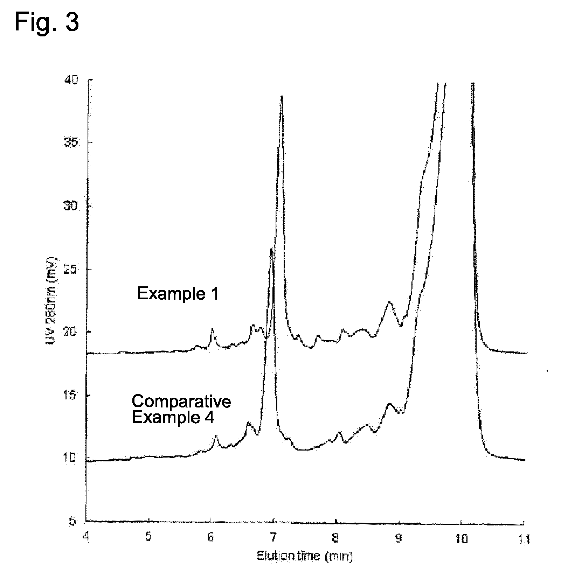

Eluent A: 20 mmol/L sodium phosphate buffer (pH 6.3)
Eluent B: 20 mmol/L sodium phosphate buffer containing 0.20 mol/L NaClO4 (pH 6.3)
Gradient: 0 to 100% eluent B linear gradient, 10 minutes
Flow rate: 0.6 mL/min
Detection: UV 415 nm
Object: HbA1c calibrator 2 (manufactured by TOSOH CORPORATION) 5 µL As shown in the chromatogram of Fig. 3, it was found that the separation of isomers was excellent as compared with Comparative Examples.

### EXAMPLE 2

A polyacrylic acid was immobilized in the same manner as in <Immobilization of polyacrylic acid> in Example 1, and then aspartic acid was introduced by the following method.

### <Introduction of aspartic acid>

4.5 g of deionized water suction dry particles were weighed, and 20 mL of ethanol water was added thereto to disperse the particles. Further, 20 mL of aqueous aspartic acid solution adjusted to have a concentration of 14 mmol/L and a pH of 7.0, and 60 mg of 4-(4,6-dimethoxy-1,3,5-triazin-2-yl)-4-methylmorpholinium=chloride n-hydrate were added thereto to carry out dispersion. The dispersion was stirred at room temperature for 1.5 hours, and then washed with deionized water by centrifugal filtration 3 times. Further, the particles were washed by centrifugal filtration with 0.5 N sodium hydroxide 3 times and with 0.5 N hydrochloric acid 3 times, respectively, and recovered as deionized water suction dry gel. The ion exchange capacity of the particles obtained was 32 µ equivalent/mL.

The cation exchanger obtained was packed in the form of slurry into a liquid chromatography column having an inner diameter of 4.6 mm and a length of 100 mm, together with a 10% deionized water as dispersion fluid. Using the column obtained, liquid chromatography was carried out under the same conditions as in Example 1 to measure the separation performance for a monoclonal antibody. The operation pressure of the cation exchanger was an operation pressure at the time of feeding eluent A at a flow rate of 0.6 mL/min.

Chromatograms were shown in Fig. 1 and Fig. 2 (an enlarged comparative diagram where elution time was corrected, together with Comparative Example 4). It was found that strong retention was achieved as compared with Comparative Examples, and it was also found that the separation performance between isomers were excellent. The elution time difference of from the elution peak 1 at an acidic side to the elution peak 2 at a basic side was 20.3 minutes, and broad selectivity was shown. The operation pressure was 17.0 MPa and was found to be low.

### EXAMPLE 3

A polyacrylic acid was introduced in the same manner as in <Immobilization of polyacrylic acid> in Example 1, and then glutamic acid was introduced by the following method.

### <Introduction of glutamic acid>

4.5 g of deionized water suction dry particles were weighed, and 20 mL of ethanol water was added thereto to disperse the particles. Further, 20 mL of an aqueous glutamic acid solution adjusted to have a concentration of 14 mmol/L and a pH of 7.0, and 60 mg of a 4-(4,6-dimethoxy-1,3,5-triazin-2-yl)-4-methylmorpholinium=chloride n-hydrate were added thereto to carry out dispersion. The dispersion was stirred at room temperature for 1.5 hours, and then washed with deionized water by centrifugal filtration 3 times. Further, the particles were washed by centrifugal filtration with 0.5 N sodium hydroxide 3 times and with 0.5 N hydrochloric acid 3 times, respectively, and recovered as deionized water suction dry gel. The ion exchange capacity of the particles obtained was 29 µ equivalent/mL.

The cation exchanger obtained was packed in the form of slurry into a liquid chromatography column having an inner diameter of 4.6 mm and a length of 100 mm, together with 10% deionized water as dispersion fluid. Using the column obtained, liquid chromatography was carried out under the same conditions as in Example 1 to measure the separation performance for a monoclonal antibody. The operation pressure of the cation exchanger was an operation pressure at the time of feeding eluent A at a flow rate of 0.6 mL/min.

As shown in the chromatogram of Fig. 1, it was found that strong retention was achieved as compared with Comparative Examples, and it was also found that the separation performance between isomers was excellent. The elution time difference of from the elution peak 1 at an acidic side to the elution peak 2 at a basic side was 25.9 minutes, and broad selectivity was shown. The operation pressure was 19.0 MPa and was found to be low.

### COMPARATIVE EXAMPLE 1

Epoxylated non-porous particles obtained in preparation of the non-porous base matrix were dispersed in water, and heated in an autoclave at 140°C for 3 hours thereby to convert epoxy groups into diol groups. 4.5 g of suction dry particles were dispersed in 8 g of deionized water, and 3.0 g of sodium α-chloroacetate was added, dissolved and dispersed therein. 2.7 g of 48 wt% sodium hydroxide was further added thereto, followed by shaking for 2 hours in a water bath set at 55°C. The particles obtained were filtrated by a glass filter, and washed with 0.1 mol/L phosphoric acid, 0.1 mol/L sodium hydroxide and deionized water in this order. The ion exchange capacity of the particles obtained was 36 µ equivalent/mL. The particles were packed into a liquid chromatography column in the same manner as in Example 1, and the separation performance for a monoclonal antibody was compared under the same measurement conditions as in Example 1.

As shown in the chromatogram of Fig. 1, the retention was weak, the peak was therefore broad, and the separation performance between isomers was poor. The elution time difference of from the elution peak 1 at an acidic side to the elution peak 2 at a basic side was 5.2 minutes, and the selectivity was lower than Example 1. The operation pressure was so low as 15.0 MPa.

### COMPARATIVE EXAMPLE 2

Using the cation exchange particles prepared in Comparative Example 1, the same operation as in <Introduction of aspartic acid> in Example 1 was carried out to obtain a cation exchanger having aspartic acid introduced. The ion exchange capacity of the particles obtained was 34 µ equivalent/mL. The particles were packed into a liquid chromatography column in the same manner as in Example 1, and the separation performance for a monoclonal antibody was compared under the same measurement conditions as in Example 1.

As shown in the chromatogram of Fig. 1, it was found that the retention was weak, and the separation performance between isomers was also insufficient. The elution time difference of from the elution peak 1 at an acidic side to the elution peak 2 at a basic side was 4.4 minutes, and the selectivity was lower than Example 1. The operation pressure was 18.0 MPa and was found to be relatively low.

### COMPARATIVE EXAMPLE 3

To an aqueous solution of 9 g of aspartic acid adjusted to have a pH of 12.8 with a 0.2 mol/L aqueous sodium hydroxide solution, 3 g of epoxylated particles obtained in preparation of the non-porous base matrix were charged, and heated in a water bath at 50°C for 2 hours to obtain a cation exchanger having aspartic acid introduced to its epoxy groups. The ion exchange capacity of the particles obtained was 42 µ equivalent/mL. The particles were packed into a liquid chromatography column in the same manner as in Example 1, and the separation performance for a monoclonal antibody was compared under the same measurement conditions as in Example 1.

As shown in the chromatogram of Fig. 1, it was found that the retention was weak, and the separation performance between isomers was also insufficient. The elution time difference of from the elution peak 1 at an acidic side to the elution peak 2 at a basic side was 5.4 minutes, and the selectivity was lower than Example 1. The operation pressure was 14.0 MPa and was found to be relatively low.

### COMPARATIVE EXAMPLE 4

The cation exchanger which underwent only the operation of <immobilization of polyacrylic acid> in Example 1, was packed into a liquid chromatography column in the same manner as in Example 1, and the separation performance for a monoclonal antibody was compared under the same measurement conditions as in Example 1. Chromatograms are as shown in Fig. 1 and Fig. 2 (enlarged comparative diagram where the elution time was corrected together with Example 2). It was found that strong retention was achieved as compared with Comparative Examples 1 and 2, and the separation performance between isomers was also improved. However, the elution time difference of from the elution peak 1 at an acidic side to the elution peak 2 at a basic side was 13.6 minutes, and it was found that the selectivity was lower than Examples 1 and 2. The operation pressure was so high as 30.0 MPa.

Further, the separation performance for glycohemoglobin was compared in the same manner as in Example 1. The chromatogram is as shown in Fig. 3, and the separation performance for isomers was insufficient.

### INDUSTRIAL APPLICABILITY

The cation exchanger of the present invention is useful as a cation exchanger for liquid chromatography in various fields, and particularly useful for separation and purification of bio samples such as various proteins including a bio body and glycohemoglobin.

The entire disclosure of Japanese Patent Application No. 2013-075020 filed on March 29, 2013 including specification, claims, drawings and summary is incorporated herein by reference in its entirety.

## Claims

1. A cation exchanger for liquid chromatography, comprising non-porous particles and, immobilized to the surface thereof, a polyacrylic acid into which a dicarboxylic acid compound having an amino group is introduced by an amide bond.

2. The cation exchanger for liquid chromatography according to Claim 1, wherein the non-porous particles are an inorganic base matrix of silica, zirconia or alumina, or an organic base matrix of a crosslinked polysaccharide or a vinyl monomer crosslinked polymer, having a volume average particle size of at most 5 µm.

3. The cation exchanger for liquid chromatography according to Claim 2, wherein the organic base matrix is a crosslinked polymer of a monofunctional vinyl monomer and a polyfunctional vinyl monomer.

4. The cation exchanger for liquid chromatography according to any one of Claims 1 to 3, wherein the polyacrylic acid has a weight average molecular weight of at least 5,000 and at most 20,000.

5. The cation exchanger for liquid chromatography according to any one of Claims 1 to 4, wherein the dicarboxylic acid compound having an amino group is aspartic acid or glutamic acid.

6. The cation exchanger for liquid chromatography according to any one of Claims 1 to 5, which has an ion exchange capacity of from 10 to 50 µ-equivalent/mL.

7. A process for producing the cation exchanger for liquid chromatography as defined in any one of Claims 1 to 6, which comprises reacting a functional group of the non-porous particles with a carboxylic acid in the polyacrylic acid thereby to immobilize the polyacrylic acid onto the surface of the non-porous particles, and reacting the immobilized polyacrylic acid with the dicarboxylic acid compound having an amino group.

8. A process for producing the cation exchanger for liquid chromatography as defined in any one of Claims 1 to 6, comprising reacting a functional group of the non-porous particles with the polyacrylic acid into which a dicarboxylic acid compound having an amino group is introduced thereby to immobilize the polyacrylic acid onto the surface of the non-porous particles.

9. A liquid chromatography column packed with the cation exchanger for liquid chromatography as defined in any one of Claims 1 to 6.

10. A method of measuring a charge isomer of an antibody by ion exchange chromatography, comprising using the liquid chromatography column as defined in Claim 9.

11. A method of measuring glycohemoglobin by ion exchange chromatography, comprising using the liquid chromatography column as defined in Claim 9.
